(19) Europäisches Patentamt
European Patent Office
Office européen des brevets

(11) EP 1 537 161 B1

(12) # EUROPÄISCHE PATENTSCHRIFT

(45) Veröffentlichungstag und Bekanntmachung des Hinweises auf die Patenterteilung:
**29.03.2006 Patentblatt 2006/13**

(21) Anmeldenummer: **03793738.0**

(22) Anmeldetag: **20.08.2003**

(51) Int Cl.:
***C08G 61/00*** (2006.01) ***C09K 11/06*** (2006.01)

(86) Internationale Anmeldenummer:
**PCT/EP2003/009199**

(87) Internationale Veröffentlichungsnummer:
**WO 2004/022626 (18.03.2004 Gazette 2004/12)**

(54) **PROZESS ZUR HERSTELLUNG VON ARYL-ARYL GEKOPPELTEN VERBINDUNGEN**

METHOD FOR THE PRODUCTION OF ARYL-ARYL COUPLED COMPOUNDS

PROCEDE DE PRODUCTION DE COMPOSES COUPLES ARYLE-ARYLE

(84) Benannte Vertragsstaaten:
**AT BE BG CH CY CZ DE DK EE ES FI FR GB GR HU IE IT LI LU MC NL PT RO SE SI SK TR**

(30) Priorität: **06.09.2002 DE 10241814**

(43) Veröffentlichungstag der Anmeldung:
**08.06.2005 Patentblatt 2005/23**

(73) Patentinhaber: **Covion Organic Semiconductors GmbH
65926 Frankfurt (DE)**

(72) Erfinder:
- **FALCOU, Aurelie
55128 Mainz (DE)**
- **SCHWAIGER, Jochen
65931 Frankfurt (DE)**
- **RITTER, Andrea
61449 Steinbach/Ts. (DE)**

(74) Vertreter: **Luderschmidt, Schüler & Partner GbR
Patentanwälte,
Industriepark Höchst,
Geb. F821
65926 Frankfurt am Main (DE)**

(56) Entgegenhaltungen:
**EP-A- 0 012 201** **EP-A- 1 229 063**
**US-A- 4 326 989**

- **K.R. CARTER ET AL.: "Self-Encapsulation of poly-2,7-fluorenes in a Dendrimer Matrix" JOURNAL OF THE AMERICAN CHEMICAL SOCIETY, Bd. 123, Nr. 9, 2001, Seiten 6965-6972, XP002265061**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Diese Erfindung bezieht sich auf die Herstellung Aryl-Aryl gekoppelter Verbindungen und Materialien. Diese Materialien spielen eine bedeutende Rolle in der Industrie, unter anderem als Flüssigkristalle, Pharmaka und Agrochemikalien, um nur einige der Anwendungsfelder zu nennen. Vor allem in dem stark wachsenden Feld organischer Halbleiter (z.B. Anwendungen in organischen bzw. polymeren Leuchtdioden, organischen Solarzellen, organischen ICs) sind gerade diese Verbindungen von herausragender Bedeutung.

Für die Synthese solcher Verbindungen sind verschiedene Alternativen bekannt, die jedoch nicht für alle Fälle eine - z.B. technisch, ökonomisch oder ökologisch - befriedigende Lösung anbieten. Bei vielen Verfahren treten Fehlprodukte bzw. Fehlreaktionen auf, die aufwendig abgetrennt und entsorgt werden müssen, oder die nicht entfernt werden können und dann zu Problemen in der Verwendung des Materials führen.

Besonders wichtig ist die Effizienz (Umsatzgrad & Selektivität) des Verfahrens, wenn es sich um die Umsetzung einer oder mehrerer multifunktioneller Verbindung(en) handelt. Ein Beispiel dieser Art von Reaktion ist die Umsetzung einer monofunktionellen Verbindung mit sich selbst, was zu einem diskreten Molekül führt. Ein weiteres Beispiel ist eine Polymerisation, wobei eine oder mehrere multifunktionelle Verbindung(en) mit einer oder mehreren weitere(n) multifunktionellen Verbindung(en) umgesetzt werden. Bei vielen Anwendungen der Polymere ist ein hohes Molekulargewicht erforderlich, um die gewünschten physikalischen Eigenschaften, z.B. Filmbildung, Flexibilität, mechanische Stabilität und weitere zu erreichen. Besonders bei organischen Halbleitern werden die elektrischen Eigenschaften stark durch das Molekulargewicht beeinflußt - meistens ist ein sehr hohes Molekulargewicht erforderlich, um u.a. Defekte wie Kurzschlüsse im elektrischen Device zu verhindern. Des weiteren können bei kurzkettigen Polymeren die unweigerlich vorhandenen Endgruppen, die dann einen relativ hohen Anteil ausmachen, wie Verunreinigungen wirken. Für diese Anwendung ist weiterhin eine hohe Reproduzierbarkeit des Prozesses erforderlich. Der Polymerisationsgrad (*DP*, durchschnittliche Zahl der Wiederholeinheiten in der Kette) eines durch schrittweises Wachstum aufgebauten Polymers hängt mit dem Umsatzgrad der Reaktion (*p*) folgendermaßen zusammen:

$$DP = \frac{1}{1-p}$$

Wenn ein hoher *DP* angestrebt wird, braucht man eine sehr effiziente Reaktion, z.B. ein $p = 0.95$ ergibt $DP = 20$ oder ein $p=0.99$ ergibt ein $DP = 100$.

Die sogenannte *Yamamoto-Reaktion* (T. Yamamoto und A. Yamamoto, *Chem.Lett.,* **1977**, 353-356 und Japanische Anmeldung JP 02-008213) hat sich als eine geeignete Reaktion zur Herstellung Aryl-Aryl gekoppelter Verbindungen erwiesen. Darunter versteht man die Homokupplung von zwei aromatischen Halogenid-Verbindungen in Gegenwart einer äquimolaren Menge eines Übergangsmetallkomplexes (in der Regel eine Ni-(0)-Verbindung, meistens Ni $(COD)_2$, welches nicht nur teuer, sondern auch giftig ist), in einer Protonen-freien Umgebung und unter inerter Atmosphäre. Es ist in der Regel üblich, die Reaktion bevorzugt mit den etwas teureren, aber deutlich reaktiveren Arylbromiden durchzuführen, diese sind synthetisch durch eine Vielzahl von Bromierungsreaktionen leicht zugänglich. Eine analoge Verwendung dieses Prozesses für eine Polymerisation ist beispielsweise in EP-A-1229063 (Sumitomo Chemicals) beschrieben. Auch hier werden wiederum äquimolare bzw. sogar überäquimolare Mengen von Ni-(0)-Komplexen verwendet, und dies auch für notwendig erachtet.

Es sind mehrere katalytische Varianten der Reaktion schon bekannt, jede weist Einschränkungen hinsichtlich ihrer ökonomischen Verwertbarkeit auf. Gründe hierfür liegen u.a. in der Substrattoleranz, der Aufreinigungsprozedur, dem Katalysatorsystem mit seinen benötigten Liganden oder der Reaktionsausbeute. Trotzdem bilden diese Arbeiten (vgl. EP-A-0012201, EP-A-0025460, WO 96/39455, WO 90106295, WO98/33836) den nächstliegenden Stand der Technik zur vorliegenden Anmeldung, und werden via Zitat als Bestandteil der Anmeldung betrachtet, um nicht alle der dort gemachten ausführlichen Beschreibungen zum allgemeinen Stand der Technik bezüglich Yamamoto-Reaktionen wiederholen zu müssen.

In den bereits von Yamamoto et al. über die Grignard-Verbindungen durchgeführten, von Übergangsmetallen katalysierten Kupplungsreaktionen müssen ganz generell gegenüber Grignard-Bedingungen instabile funktionelle Gruppen wie Ketone und Ester vermieden werden. (Japanische Anmeldung JP 52-154900). Diese Einschränkung hinsichtlich der Substratauswahl limitiert die Zahl an nutzbringenden Reaktionen beträchtlich. Eine Variante dieses Prozesses wird mit Zink anstelle des Magnesiums beschrieben. (Japanische Anmeldung JP 61-233014)

Die substrattolerantere Variante dieser Reaktion wurde von Colon et al. vorgestellte (I. Colon und D.R. Kelsey, *J. Org.Chem.,* 51, **1986**, 2627-2637, Europäische Anmeldungen EP-A-0025460 und EP-A-0012201). Hierbei wird eine Nickelkomponente *in situ* mit Zinkpulver in Gegenwart eines Phosphins (bevorzugt Triphenylphosphin) und eines bidentaten Liganden reduziert, um daraus einen aktiven Nickel(0)phosphinkatalysator herzustellen. Ohne hier näher auf die Theorie des Mechanismus eingehen zu wollen, wird angenommen (siehe u.a. C.A. Tolman, W.C. Seidel und L.W. Gosser, J. *Am. Chem. Soc.*, 96, **1974**, 53), daß die Nickelspezies im katalytischen Zyklus einen oder mehrere ihrer Phosphinliganden verliert und sich koordinativ ungesättigte Komplexe bilden. In manchen der katalytischen Teilschritte sind die ungesättigten Ni-Komplexspezies essentiell für die Reaktivität der Arylhalogenide, gleichzeitig aber in anderen Teilschritten auch Ursache für die Bildung unerwünschter Nebenprodukte. Bei hohen Konzentrationen an koordinativ ungesättigten Komplexen können sich daraus inerte Komplexe bilden, die keine katalytische Wirkung mehr zeigen. Eine zweite unerwünschte Nebenreaktion ist die Abstraktion einer Phenylgruppe des Phosphinliganden und daraus resultierende, unerwünschte Kupplungsprodukte mit den Arylhalogeniden. Sie vermindern die Effizienz der Reaktion und erhöhen den Reinigungsaufwand zur Gewinnung des erwünschten Produktes. Durch eine Erhöhung der Phosphinkonzentration in der Reaktionsmischung kann diese Nebenreaktion verringert werden.

Die Konzentration des Phosphinliganden, bevorzugt Triphenylphosphin und ähnliche Triarylphosphine, sollte zwischen 1 und 30 Äquivalenten der Nickelmenge liegen, wobei auf Grund praktischer Erfahrungen zur Minimierung beider obengenannter Effekte (siehe: Wang et al., WO 96/39455) eine Bandbreite der Konzentration von 0,1 bis 0,5 mol/L für den Phosphinligenden vorteilhaft ist (bei Konzentrationen von 0,5 bis 1 mol/L für das Arylhalogenid und 0,01 bis 0,05 mol/L für Nickel). Unter diesen Bedingungen ist die Bildung unerwünschter Kupplungsprodukte aber nicht unterdrückt und die hohen Phosphinzugaben machen im Anschluß aufwendige Reinigungsschritte notwendig.

Ein bidentater Ligand (2,2'-Bipyridin, 1,10-Phenanthrolin etc.), etwa 0,2 - 2 Äquivalente im Vergleich zur eingesetzten Nickelmenge, kann der Reaktionsmischung beigemengt werden, größere Mengen werden auf Grund seiner chelatisierenden Wirkung als nicht vorteilhaft beschrieben (Colon et al., *J. Org. Chem.,* 51, **1986,** 2627-2637). Darüber hinaus kann ein Promoter aus der Gruppe der Alkali-, Erdalkali-, Zink-, Magnesium- und Aluminiumhalogenide, -sulfate oder -phosphate im Bereich von 1 bis 100 Äquivalenten der eingesetzten Nickelmenge zugesetzt werden. Als reduzierendes Metall wird Zink, Magnesium oder Mangan, ersteres bevorzugt, in äquimolaren Mengen des Arylhalogenids eingesetzt. Die Grenzen der Yamamotoreaktion in der Variante nach Colon liegen bei Substraten mit zwei ortho-ständigen Substituenten zum Halogenid, einfach orthosubstituierte Halogenide zeigen ebenfalls geringere Ausbeuten.

In dem Versuch, die Bildung der Nebenprodukte zu minimieren und das Aufreinigungsproblem zu lösen, sind weitere Verfahren entwickelt worden. In der Anmeldeschrift WO 96/39455 beschreiben Wang et al. ein Kupplungsverfahren zur Umsetzung von Arylhalogeniden (bevorzugt Chloriden) oder Arylsulfonaten zur Herstellung von Biarylen und Polyarylen. In diesem Verfahren kommt eine Katalysatormischung aus einer Nickelkomponente, einem reduzierenden Metall (bevorzugt aktivierter Zinkstaub) und einem Phosphitliganden zur Anwendung. Das Verhältnis des Liganden zum Nickel liegt dabei zwischen 1 und 10 Äquivalenten. Dieser Prozeß weist jedoch ebenfalls mehrere Schwachstellen auf, von denen im Folgenden einige genannt werden:

Erstens müssen die benötigten Phosphitliganden aufwendig synthetisch dargestellt werden und sind aus der Reaktionsmischung nach der Aufarbeitung nicht rückgewinnbar.

Zweitens hat die chemische Struktur des Phosphitliganden einen gravierenden Einfluß auf die Stabilität der Nickel(O) komplexe im katalytischen Prozeß und muß für unterschiedliche Substrate neu optimiert werden.

Drittens findet bei reaktionsträgeren Edukten mit elektronenschiebenden Substituenten in *ortho-* oder *para-*Stellung in größerem Ausmaß eine störende Nebenreaktion, nämlich die nickelkatalysierte Substitution des Halogens durch Wasserstoff, statt.

Viertens entsprechen die erzielten Molekurargewichte $M_w$ der Polymer-Produkte einem Polymerisationsgrad DP in der Größenordnung von DP < 100.

Aus den oben genannten Gründen folgt, daß die beschriebene Reaktion für einen effizienten Polymerisationsprozeß zur Darstellung hoher Molekulargewichte nicht geeignet ist und die Verwendung von käuflich nicht erhältlichen Liganden eine besondere Erschwemis darstellt.

In der Anmeldeschrift WO 90/06295 der Firma Eastman Kodak wird ein Verfahren zur Herstellung von biarylischen Komponenten aus Arylchloriden vorgestellt. Die Anwendung von bidentaten Organophosphinen als Liganden wird beschrieben. Das Katalysatorsystem besteht darüber hinaus aus einer Nickelspezies (im Verhältnis von 1:3 Äquivalenten zum Phosphinliganden) und einem reduzierenden Metall wie Zink oder ähnlichem in einem wasserfreien, dipolaren, aprotischen Medium. Optional können dem phosphorhaltigen Liganden äquimolare Mengen eines stickstoffhaltigen Liganden wie Bipyridin sowie anorganische Salze im Bereich von 1 bis 100 Äquivalenten der eingesetzten Nickelmenge zugesetzt werden.

Auch dieses Verfahren ist technisch nur bedingt anwendbar. Wiederum müssen die bidentaten Phosphinliganden substratspezifisch gewählt und dargestellt werden. Die Bildung von größeren Mengen an dehalogeniertem Edukt bei der reduktiven Dimerisierung von substituierten Arylhalogeniden, im Durchschnitt zwischen 5 und 15 Prozent, in einigen Fällen bis zu 60 Prozent, stellt den wohl größten Nachteil dieses Verfahrens dar. Darüber hinaus kommt es häufig zur Bildung von isomer verknüpften Dimeren. Die Darstellung von polymeren Verbindungen ist mit diesem Verfahren nicht beschrieben, und sollte wegen der o. g. Probleme auch nicht gelingen.

In der Anmeldeschrift WO 98/33836 beschreiben Aiike et al. die Kupplungsreaktion zwischen substituierten

Sulfonyloxyphenylen selbst oder diversen anderen Monomeren mit Hilfe eines Katalysatorsystems, bevorzugt bestehend aus einem Nickelmetallsalz und einem Liganden wie Triphenylphosphin oder 2,2'-Bipyridin. Diese Liganden können individuell oder in Kombination zugesetzt werden. Als Reduktionsmittel im katalytischen System werden Zink und Mangan bevorzugt eingesetzt. Ein anorganisches Salz wie NaBr, NaI oder Tetraethylammoniumiodid kann zugesetzt werden. Diese Patentschrift beschränkt sich allerdings auf die genannten Sulfonyloxyphenylenprodukte.

Aus dieser Kritik am Stand der Technik ist es klar, daß es immer noch Bedarf an hoch-effizienten Verfahren gibt, die bei geringer Katalysator-Konzentration zu Aryl-Aryl gekoppelten Verbindungen, mit möglichst wenigen Fehlreaktionen führen. Zur eindrücklicheren Darstellung sind repräsentative Beispiele aus den genannten Schriften in der folgenden Tabelle zusammengefaßt.

| | Zitat 1 | Zitat 2 | Zitat 3 | Zitat 4 |
|---|---|---|---|---|
| | EP 12201 | WO 96/39455 | WO 90/06295 | WO 96/39455 |
| Edukte | p-Chloranilin | 4-Chlortoluol | 2-Chlortoluol | 2,5-Dichlorbenzophenon |
| Lösungsmittel | DMAC | NMP | DMF | NMP |
| Temperatur | 80°C | 60°C | 70°C | 90°C |
| Konzentration an Edukt | 1.57 mol/L | 1.58 mol/L | 1.33 mol/L | 0.96 mol/L |
| Molares Verhältnis von Edukt zu Nickelspezies | 1:0.06 | 1 : 0.05 | 1 : 0.05 | 1 : 0.075 |
| Molares Verhältnis von Edukt zu Liganden | 1:0.5 | 1:0.1 | 1:0.075 mit zwei Phosphoratomen pro Ligand | 1:0.4 |
| Reaktionszeit | 1h | 17h | 16h | 36h |
| Ausbeute | 88% (GC) | 96% (GC) | 95% (GC) | 92% |
| $M_w$ [g/mol] | Dimer | Dimer | Dimer | 14,319 |

*Tabelle 1 Literaturvergleich von bekannten Darstellungsverfahren.*

Wir haben nun überraschend festgestellt, daß bei Verwendung von Ni-(0)-Komplexen, die eine spezielle Liganden-Anordnung aufweisen, und der Verwendung eines Reduktionsmittels mit sehr niedrigen, also katalytischen Mengen Ni ein besonders effizientes Yamamoto-Verfahren erreicht wird.

Gegenstand der Erfindung ist somit ein Verfahren zur Kupplung aromatischer oder heteroaromatischer Halogenverbindungen unter Bildung einer oder mehrerer C-C-Einfachbindung, dadurch gekennzeichnet,
daß ein Ni-(0)-Komplex, welcher mindestens zwei verschiedenartige Liganden enthält, wobei jeweils mindestens ein Ligand aus jeder der beiden Gruppen der heteroatom-haltigen Liganden (Gruppe 1) und der $\pi$-System Liganden (Gruppe 2) gewählt werden in katalytischen Mengen verwendet wird,
und ein Reduktionsmittel zum Einsatz kommt, welches verbrauchtes Nickel wieder in Ni-(0) überführt;
die Reaktion in einem wasserfreien, aprotischem Medium unter weitestgehend inerter Atmosphäre stattfindet,

mit der Maßgabe, daß dabei keine Phosphor-haltige Verbindung zugesetzt wird.

Die erfindungsgemäße Reaktion kann nun (je nach genauer Zusammensetzung und Temperatur) entweder ein- oder auch mehrphasig ablaufen, bzw. dies auch während der Reaktionsdurchführung ändern. Bevorzugt läuft die erfindungsgemäße Reaktion jedoch einphasig ab.

Aryl- bzw. Heteroarylverbindungen sind Aromaten bzw. Heteroaromaten mit 2 bis 40 C-Atomen, welche mit einem oder auch mehreren linearen, verzweigten oder cyclischen Alkyl- bzw. Alkoxyresten mit 1 bis 20 C-Atomen, bei denen wiederum eine oder mehrere $CH_2$-Gruppen, die nicht aufeinander folgen, durch O, C=O, oder eine Carboxygruppe ersetzt sein können, substituierten oder unsubstituierten C-2 bis C-20 Aryl- oder Heteroarylresten, Fluor, Cyano-, Nitrogruppen substituiert sein können bzw. auch unsubstituiert sein können. Einfache Verbindungen, die bevorzugt verwendet werden können, sind die entsprechenden substituierten oder unsubstituierten Derivate von Benzol, Naphthalin, Anthracen, Pyren, Biphenyl, Fluoren, Spiro-9,9'-bifluoren, Phenanthren, Perylen, Chrysen, Naphthacen, Pentacen, Triptycen, Pyridin, Furan, Thiöphen, Benzothiadiazol, Pyrrol, Chinolin, Chinoxalin, Pyrimidin und Pyrazin.

Weiterhin sind ausdrücklich entsprechende (im Sinne des obigen Textes) multifunktionelle Verbindungen mit umfaßt, ebenso die bei einer Polymerisation auftretenden Oligomeren mit funktionellen Aryl- bzw. Heteroaryl-Enden.

Die Ausgangsverbindungen für das erfindungsgemäße Verfahren sind aromatische oder heteroaromatische Halogenverbindungen der Formel (I),

$$Ar\text{-}(X)_n \qquad (I)$$

worin Ar eine Aryl- bzw. Heteroarylverbindung wie oben definiert ist, X -Cl, -Br, -I bedeutet und n mindestens 1, bevorzugt 1 bis 20, besonders bevorzugt 1, 2, 3, 4, 5 oder 6 bedeutet, aber maximal den Wert beträgt, welcher der Anzahl aromatischer Protonen entspricht, die in der Ausgangsverbindung vorliegen können.

Für die Darstellung linearer Polymere wird nun bevorzugt für n für alle verwendeten Monomere der Wert 2 gewählt.

Für das erfindungsgemäße Verfahren kommen Ni-(0)-komplexe zum Einsatz. Diese können entweder direkt gekauft werden ($Ni(COD)_2$ u. ä.), in-situ aus entsprechenden Vorstufen oder Maßlösungen erzeugt werden, bevorzugt aber kann die Katalysatorlösung vorab hergestellt werden.

Für die Herstellung entsprechender Ni-(0)-komplexe kommen nun u. a. folgende Ni-Verbindungen in Frage: elementares Nickel bzw. auch disperses oder kolloidales metallisches Nickel, geträgert oder ungeträgert, wie Nickelschwamm, Nickel auf Kieselgur, Nickel auf Aluminiumoxid, Nickel auf Silica, Nickel auf Kohle, Nickel(II)acetat, Nickel (II)acetylacetonat, Nickel(II)chlorid, -bromid, iodid, Nickel-(II)-carbonat, -ketonate, -formiat, -sulfat, oder daraus ableitbare Komplexe wie Olefinnickel-(II)-halogenide, Allylnickel-(II)-halogenide, Additionsverbindungen des Typs $NiL_2X_2$ wobei X Chlor, Brom, Iod und L einem neutralen Liganden wie z. B. Ammoniak, Acetonitril, Propionitril, Benzonitril, entspricht, Nickel(II)nitrat, Nickel(II)sulfat, Nickel(II)oxalat, Bis-cyclooctadiennickel(0), Tetrakistriphenylphosphinnickel(0) oder auch weitere Nickel-(0)-verbindungen.

Wie oben ausgeführt kann die Ni-(0)-spezies bevorzugt vorab in einer Katalysatorlösung bereitet werden. Für die Herstellung der Katalysatorlösung wird dabei im Allgemeinen wie folgt vorgegangen:

Ein Reduktionsmittel (z. B. Mn) wird mit, beispielsweise in DMF gelösten Ni-(11)-salz (z. B. $NiBr_2$), bei Raumtemperatur in einer inerten Atmosphäre gemischt. Die Liganden-Lösung (z. B. Bipyridyl und COD in Toluol gelöst) wird langsam zugegeben, nach 5-10 Minuten wird die Lösung tief violett. Diese wird eine Nacht bei Raumtemperatur kräftig gerührt. Die Lösung ist einige Wochen unter geeigneten, trockenen Schutzgasen stabil und sollte bevorzugt unter Schutzgasatmosphäre gelagert und manipuliert werden.

Die beschriebene Darstellung einer Ni-(0)-Komplexlösung ist ebenfalls Gegenstand der Erfindung.

Als Liganden werden nun - gemäß den o. g. Angaben - zum einen solche aus der Gruppe 1 benutzt. Diese sind wie folgt definiert:

- Allgemein sind dies Liganden, die eine über ein Heteroatom verlaufende $\eta^1$-Koordination zum Nickel aufweisen. Bevorzugt handelt es sich dabei um Heteroatome der 5. und 6. Hauptgruppe, allerdings unter Ausnahme von Phosphor, besonders bevorzugt um Stickstoff und/oder Sauerstoff. Im allgemeinen kann es sich hierbei um aliphatische Verbindungen (linear bzw. verzweigt) oder aliphatische bzw. aromatische Cyclen handeln, bevorzugt jedoch um mono-, bi- oder tricyclische Ringsysteme.
- Bevorzugt ist jedoch, wenn die Liganden der Gruppe 1 zweizähnig sind, d. h. zwei $\eta^1$-Koordinationen zum Nickel aufweisen, die dabei jeweils über die Heteroatome verlaufen. Dies ist der Fall, wenn die Liganden in der Lage sind, das im folgenden Schema bezeichnete Bis-$\eta^1$-X,Y-koordinierte Komplexfragment zu bilden, wobei X und Y bevorzugt gleich oder verschieden Stickstoff oder Sauerstoff sind.

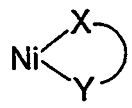

(wobei der dargestellte Halbkreis eine im Text beschriebene Alkyl- oder Arylbrücke darstellt).
Als einzelne Beispiele seinen die folgenden angeführt:

1. Für den Fall, daß X und Y Stickstoff sind, wären dies Liganden vom Typ des 2,2'-Bipyridins, 1,10-Phenanthrolins, 1,1'-Bisisochinolins, 2,2'-Bichinolins,1,8-Diazabicyclo[5.4.0]undec-7-en u.ä. Ebenso sind damit Liganden vom Typ der, in dem nachstehenden Schema beispielhaft vorgestellten, an 2-Position Methanamin- oder Imidoyl-substituierten Pyridine wie z.B. das α-Ethyl-α-methyl-N,N-[di-*iso*-propyl]-2-pyridinmethanamin oder das 2-(N-Methylacetimidoyl)-pyridin u. ä. gemeint.

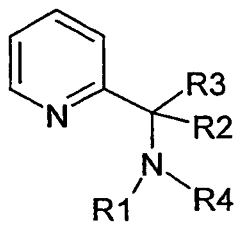

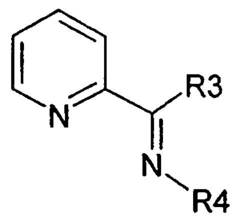

(R1, R2, R3 und R4 können unabhängig voneinander z. B. ein Wasserstoffatom, Alkyl- oder Arylrest sein, wobei auch der Pyridinring als solcher noch weiter substituiert sein kann.)
2. Für den Fall das X Stickstoff und Y Sauerstoff sind, wären dies beispielsweise Liganden vom Typ der im nachstehenden Schema beispielhaft vorgestellten, an 2-Position 1-Alkoxyalkyl- oder Carbonyl-substituierten Pyridine wie z.B. 2-(1-Methoxy-1-methylethyl)-pyridin oder 2-Acetylpyridin oder 2-Pyridincarbaldehyd u. ä.

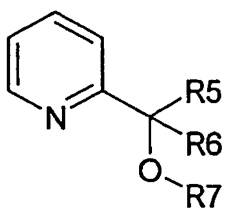

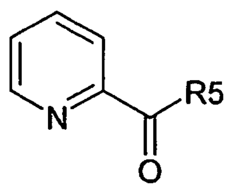

(R5, R6 und R7 können z. B. ein Wasserstoffatom, Alkyl- oder Arylrest sein, wobei auch der Pyridinring als solcher noch weiter substituiert sein kann.) Ebenso sind damit Liganden vom Typ des substituierten oder unsubstituierten 8-Alkoxychinolins gemeint.
3. Für den Fall das X und Y Sauerstoff sind, wären dies z. B. Liganden vom Typ der im nachstehenden Schema vorgestellten, an 2-Position 1-Alkoxyalkyl- oder Carbonyl-substituierten Furane wie z.B. 2-(1-Ethyl-1-methoxypropyl)-furan oder 2-Acetylfuran oder 2-Furaldehyd u. ä.

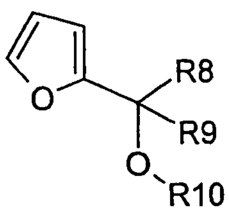

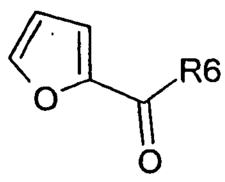

(R8, R9 und R10 können z. B. ein Wasserstoffatom, Alkyl- oder Arylrest sein, wobei auch der Furanring als solcher noch weiter substituiert sein kann.)

4. Für den Fall das X Sauerstoff und Y Stickstoff sind, wären dies die aus den oben beschriebenen Liganden abgeleiteten, im nachstehenden Schema beispielhaft vorgestellten N-Alkyl- oder N-Arylimin- und -aminderivate.

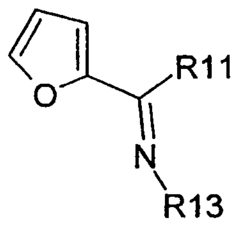

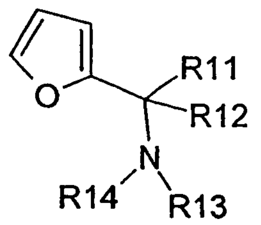

(R11, R12, R13 und R14 können unabhängig voneinander z. B. ein Wasserstoffatom, Alkyl- oder Arylrest sein, wobei auch der Furanring als solcher noch weiter substituiert sein kann.)

5. Ebenfalls sind damit Liganden vom Typ der vicinalen, N- bzw. O-difunktionalisierten Alkene gemeint, wie sie in nachfolgendem Schema exemplarisch dargestellt sind.

R15, R16, R17, R18, R19 und R20 können unabhängig voneinander z. B. ein Wasserstoffatom, Alkyl- oder Arylrest sein.

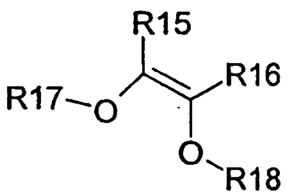

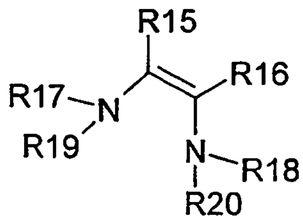

Des weiteren werden noch Liganden aus der Gruppe 2 verwendet. Diese sind wie folgt definiert:

- Allgemein sind dies Liganden, welche eine über ein π-System verlaufende $\eta^2$-Koordination zum Nickel aufweisen. Bevorzugt handelt es sich bei dem π-System dabei um Alkin-, Alken-, $\eta^2$-koordinierte Imin- oder $\eta^2$-koordinierte Carbonylgruppen, besonders bevorzugt um eine Alkin- oder Alkengruppe, ganz besonders bevorzugt um eine Alkengruppe. Im allgemeinen kann es sich hierbei um aliphatische Verbindungen, welche linear, verzweigt oder zyklisch sind, in welchen des weiteren auch einige $CH_2$-Gruppen durch einzelne Heteroatome (wie Sauerstoff, d. h. z. B. Etherbrücken) ersetzt sein können, und welche das entsprechende π-System enthalten, handeln; bevorzugt jedoch um mono-, bi- oder trizyklische Ringsysteme.
- Bevorzugt ist jedoch, wenn auch die Liganden der Gruppe 2 zweizähnig sind, d. h. zwei $\eta^2$-Koordinationen zum Nickel aufweisen, die dabei jeweils über die π-Systeme verlaufen. Dies ist der Fall, wenn die Liganden in der Lage sind, das im folgenden Schema bezeichnete Bis-$\eta^2$-A,B-koordinierte Komplexfragment zu bilden, wobei A und B zwei gleichen oder verschiedenen π-Systemen entsprechen.

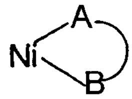

(wobei der dargestellte Halbkreis eine im weiteren Text beschriebene Überbrückung darstellt).
Als Überbrückung können beispielsweise folgende Strukturen Verwendung finden:

1. Kürzere Alkylfragmente (linear, oder verzweigt), welche aber auch noch einzelne Heteroatome enthalten können (bspw. als Ethergruppierung). Bevorzugt ist hierbei, wenn die π-Systeme durch ein bis drei, besonders bevorzugt zwei $CH_2$-Gruppen, oder äquivalente Überbrückungseinheiten, voneinander getrennt sind.

2. Zyklischen Systeme, beispielsweise mono-, bi- bzw. tricyclische Ringsysteme mit 6 bis 14-C-Atomen, von denen einzelne, wie oben beschrieben, noch durch Heteroatome ersetzt sein können. Ganz besonders geeignet sind

substitutierte oder unsubstituierte Ringe ausgewählt aus den Verbindungstypen 1,4-Cyclohexadien, Norbomadien, Bicyclo[2.2.2]octa-1,4-dien, 1,4-Cycloheptadien, 1,5-Cyclooctadien, .1,5-Cyclononadien, verschieden *cis*- bzw. *trans*-Dekalin-diene, bevorzugt Cyclooctadien-derivate.

Gegenstand der Erfindung ist nun, daß in den verwendeten aktiven Ni-(0)-komplexen je mindestens ein Ligand aus der 1. Gruppen und mindestens ein Ligand aus der 2. Gruppe enthalten ist.
Für das erfindungsgemäße Verfahren wird die Ni-(0)-Verbindung in katalytischen Mengen eingesetzt. Dies bedeutet, daß weniger als 50 mol% Ni-Verbindung (bezogen auf die Menge an Halogenid), bevorzugt weniger als 5 mol%, für Dimerisierungsreaktionen ganz besonders bevorzugt weniger als 3 mol% verwendet werden. Um eine ausreichende Reaktionsgeschwindigkeit bei Polymerisationen zu erreichen, ist es aber empfehlenswert, keine zu geringe Ni-(0)-konzentration zu verwenden. So stellen 5 mol% Ni-Verbindung eine sinnvolle Untergrenze bei Polymerisationen dar.
Um das erfindungsgemäße Verfahren auszuführen, benötigt man - wie oben beschrieben - ein Reduktionsmittel, welches "verbrauchtes" Nickel-(II) wieder in Ni-(0) rückreduziert.
Als Reduktionsmittel kommen alle Elemente bzw. Verbindungen der elektrochemischen Reihe mit einem negativeren Redoxpotential als $Ni^{2+}$ (-0,257 V) in Frage, bevorzugt Aluminium, Mangan, Eisen, Zink, Natrium, Calcium, Magnesium, ganz besonders bevorzugt Mangan-Pulver der Reinheit 99,99%. Ebenfalls möglich sind auch komplexere Reduktionsmittel, wie Metallhydride, Hydrazin und weitere organische und anorganische Reduktionsmittel.
Dieses Reduktionsmittel wird in einem stöchiometrischen Verhältnis (bezogen auf die Menge an Halogenid) im Bereich von 0.5 bis 10 Äquivalenten, ganz besonders bevorzugt im Bereich von 2 bis 6 Äquivalenten eingesetzt.
Das erfindungsgemäße Verfahren findet bevorzugt in Lösung statt. Bei geeigneten Reaktanden (d. h. solchen bei denen sowohl die Edukte, als auch die Produkte bzw. die Mischung aus Edukt und Produkt im gewählten Reaktionsbereich [p, T] flüssig sind), kann auch das/die Edukt(e) als solches dienen.
Geeignete Lösemittel sind inerte, aprotische Lösungsmittel, bevorzugt weniger polare Lösungsmitteln wie z. B. aliphatischen und aromatischen Kohlenwasserstoffen, wie Pentan, Cyclohexen, Toluol, Xylol u. ä. oder gesättigte, offenkettige, beziehungsweise zyklische Ether, wie Diethylether oder Tetrahydrofuran, besonders bevorzugt aromatische Kohlenwasserstoffe, ganz besonders bevorzugt Toluol. Diese können mit inerten, dipolaren Lösungsmitteln wie N,N'-Dimethylformamid, N,N'-Dimethylacetamid, N-Methylpyrrolidin-2-on, Tetramethylharnstoff, Dimethylsulfoxid oder Sulfolan und ähnlichem in jedem Mischungsverhältnis gemischt werden, wie z. B. in der besonders geeigneten Mischung von DMF zu Toluol in einem Verhältnis von ≤ 1:20.
Das erfindungsgemäße Verfahren liefert mit den beschriebenen katalytischen Mengen die in den unten angefügten Beispielen genannten guten Ausbeuten, wenn Sauerstoff und protische Verbindungen, insbesondere Wasser und Alkohole, weitestgehend ausgeschlossen sind.
So ist es sinnvoll, den Sauerstoffgehalt in der Reaktionsatmosphäre unter 1 %, bevorzugt unter 1000 ppm, besonders bevorzugt unter 10 ppm, ganz besonders bevorzugt unter 1 ppm zu halten.
Bezüglich protischer Verbindungen (wie zum Beispiel Wasser) gilt analoges.
Diese Reaktionsbedingungen werden v. a. durch erreicht, indem die Reaktion mit einem entsprechenden Sauerstoff-freiem bzw. -armen Schutzgas, wie Argon oder Stickstoff durchgeführt werden, bevorzugt mit Argon, bzw. das / die Lösemittel und Reaktanden vorher damit bereits gesättigt werden. Dies geschieht bevorzugt durch mehrmaliges Entgasen der Lösungsmittel und / oder Durchleiten von Schutzgas durch das Lösungsmittel.
Um protische Verunreinigungen und Wasser zu vermeiden bzw. den Gehalt auf die o. g. Werte zu bringen, werden entweder entsprechende Qualitäten eingesetzt, oder die Lösemittel durch entsprechende Literaturverfahren (siehe z. B. "Purification of Laboratory Chemicals", 2nd Edition, Pergamon Press Ltd., 1980) getrocknet bzw. gereinigt.
Bevorzugt sollte dann die Reaktion in entsprechend geeigneten, ganz besonders bevorzugt in zuvor gut mit trockenem Schutzgas, wie z.B. Argon, gefluteten Apparaturen durchgeführt werden.
Bevorzugt sollten die Edukte, die Reaktionsmischung und die Produkte unter Schutzgasatmosphäre gelagert, verarbeitet bzw. manipuliert werden, ganz besonders bevorzugt aber die Reagenzien Mangan und Bipyridin bzw. die diesen entsprechenden Reagenzien, sowie natürlich auch die gegebenenfalls daraus vorbereitete Katalysatorlösung. Gleiches gilt für die Lösungen der synthetisierten Polymere. Für das Arbeiten im Labormaßstab kann es dabei durchaus sinnvoll sein, die Arbeiten in einer Glovebox auszuführen.
Das erfindungsgemäße Verfahren ist in der Regel nur schwach exotherm und benötigt meist eine leichte Aktivierung. Häufig wird das Verfahren deshalb bei Temperaturen oberhalb von Raumtemperatur durchgeführt. Ein bevorzugter Temperaturbereich ist deshalb der Bereich zwischen Raumtemperatur und dem Siedepunkt der Reaktionsmischung, besonders bevorzugt der Temperaturbereich zwischen 40 und 120°C, ganz besonders bevorzugt der Bereich zwischen 40 und 60°C. Es kann aber auch sein, daß die Reaktion schon bei Raumtemperatur genügend schnell verlaufen, so daß keine aktive Erwärmung benötigt wird.
Die Reaktion erfolgt unter Rühren, wobei je nach Viskosität der Reaktionsmischung einfache Rührer oder Hochviskositätsrührer angewandt werden können. Bei hohen Viskositäten können auch Stromstörer verwendet werden.
Die Konzentration der Reaktionskomponenten hängt nun sehr stark von der jeweiligen Reaktion ab. Während

man Polymerisationen üblicherweise (wegen der dabei auftretenden Viskositätserhöhung) bei Konzentrationen im Bereich von weniger als 0,5 mol/L (bezogen auf zu bildende C-C-Bindungen) durchführt, kann dies bei der Synthese von definierten Einzelmolekülen auch in einem höheren Konzentrationsbereich geschehen.

Die Reaktionszeit ist prinzipiell frei wählbar und wird sich an der jeweiligen Reaktionsgeschwindigkeit orientieren. Ein technisch sinnvoller Rahmen ist im Bereich von wenigen Minuten bis zu 120 Stunden, für Dimerisierungsreaktionen bevorzugt im Bereich von 60 Minuten bis 48 Stunden zu sehen, kann bei reaktionsträgen Edukten oder Polymerisationsreaktionen diese aber übersteigen.

Die Reaktion an sich läuft unter Normaldruck ab. Technisch kann es aber auch durchaus sinnvoll sein, unter erhöhtem oder erniedrigtem Druck zu arbeiten. Dies hängt sehr stark von der Einzelreaktion und vom zur Verfügung stehenden Equipment ab,

Die Vorteile des beschriebenen erfindungsgemäßen Verfahrens sind u. a. die folgenden:

Herausragende Effizienz (Umsatzgrad), wodurch Materialien entstehen, die sehr wenige Fehlstellen, die das Ergebnis von Fehlreaktionen sind, enthalten. Besonders bei multifunktionellen Verbindungen, ist das erfindungsgemäße Verfahren von Vorteil, denn der Effizienzgrad ist dann potenziert. Ganz besonders bei Polymerisationen, wobei die Edukte im Falle linearer Polymere zwei reaktive Gruppen enthalten und die mehrmals hintereinander stattfindende Aryl-Aryl-Kupplung zur Bildung eines kettenförmigen Moleküls führt, zeigen die Produkte des erfindungsgemäßen Verfahrens außergewöhnlich hohe Kettenlängen und Molekulargewichte.

Ein besondere Vorteil der vorliegenden Erfindung ist, daß wegen der verbesserten Effizienz der Yamamoto-Reaktion bei der Polymerisation die eingesetzte Menge an Ni-(0)-Verbindung trotzdem geringer gehalten werden kann. Dies hat zur Folge, daß der Prozeß ökonomisch wie ökologisch vorteilhaft ist, und zusätzlich die Restmenge an Nickel im Produkt geringer ist. Dies bringt technische Vorteile, z.B. Vermeidung von Beeinträchtigung der Farbe des Produkts, aber besonders bei organischen Halbleitern ist die Verringerung von solchen Verunreinigungen vorteilhaft, denn die Anwesenheit von Metallresten führt zu Beeinträchtigungen in der Anwendung. I. d. R. gelingt es dabei durch einfache Aufreinigungsprozeduren, den Rest-Nickel-Gehalt auf jeden Fall unter 1000 ppm, meist auch unter 100 ppm und sehr häufig unter 10 ppm bzw. gar unter 1 ppm zu drücken. Dies ist ein herausragendes Kennzeichnen des erfindungsgemäßen Verfahren, denn die so erzeugten Produkte heben sich dadurch vom Stand der Technik deutlich ab.

Darüber hinaus werden in dem erfindungsgemäßen Verfahren vor allem die beträchtlichen Verunreinigungen durch die Abfallprodukte der P-haltigen Liganden vermieden. Die Vermeidung der bekannt aufwendigen Reinigungsprozeduren bei der Verwendung von P-haltigen Liganden stellt eine weitere maßgebliche Verbesserung des erfindungsgemäßen Verfahrens gegenüber dem derzeitigen Stand der Technik dar. Vor allem führt die Vermeidung dieser P-haltigen Liganden dazu, daß im Produkt keine P-haltigen Verunreinigungen (von den Liganden stammend) enthalten sind. Dies gilt v. a. bei Polymeren, denn hier führen Verfahren gem. dem o. g. Stand der Technik häufig dazu, daß eventuell sogar Phosphin-haltige Gruppen (als Endgruppen) ins Polymer übertragen werden, und sich dadurch der Aufreinigung entziehen. Die so erhaltenen Polymere zeichnen sich also durch ihre Phosphorfreiheit gegenüber dem Stand der Technik aus. Der Gehalt an Phosphor in dem Polymer beträgt weniger als 10ppm, vorzugsweise weniger als 1 ppm.

Die Isolierung des Produktes aus der Reaktionsmischungen erfolgt erfindungsgemäß wie in den repräsentativ angeführten Beispielen bevorzugt durch Abfiltrieren der Reaktionsmischung über Celite und darauffolgende Extraktion mit 1 M HCL oder andere geeignete Aufarbeitungsmethoden. Die erzeugten Produkte können dann durch Standardverfahren wie Umkristallisation, Destillation bei Normal- oder Unterdruck, Aus- bzw. Umfällen, Chromatographie, Sublimation u. ä. weiter gereinigt werden.

Da das erfindungsgemäße Verfahren - w. o. beschrieben - eine sehr hohe Effizienz aufweist, ist eine bevorzugte Ausführung die Umsetzung von multifunktionellen Molekülen zu Polymeren.

Multifunktionell im Sinne dieser Anmeldung soll bedeuten, daß eine Verbindung mehrere (z. B. zwei, drei, vier, fünf, usw.) gleiche oder gleichartige funktionelle Einheiten enthält, die in der entsprechenden Umsetzung (hier Yamamoto-Reaktion) alle in der gleichen Weise zu einem Produktmolekül reagieren. Mit der Umsetzung von multifunktionellen Verbindungen ist hier zunächst die Umsetzung zu einem Produkt mit polymeren Charakter gemeint. Auch dies stellt ausdrücklich eine Yamamoto-Reaktion im Sinne dieser Erfindung dar.

Polymerer Charakter liegt gemäß der vorliegenden Erfindung dann vor, wenn beim Hinzufügen bzw. Weglassen einer einzelnen Wiederholeinheit, sich die maßgeblichen Eigenschaften (z. B. Löslichkeit, Schmelzpunkt, Glastemperatur, etc.) nicht oder nur unwesentlich ändern. Eine einfachere Definition ist die Angabe des Polymerisationsgrades DP, wonach dann "polymerer Charakter" bei einem Polymerisationsgrad in der Größenordnung von DP > 100 zu definieren ist, darunter spricht man von Oligomeren.

Die durch die Verwendung des erfindungsgemäßen Verfahrens erzeugten Polyarylene (dieser Begriff soll hier auch Copolymere, welche zusätzlich Nicht-Arylen- bzw. -heteroarylen-Einheiten in der Hauptkette enthalten, umfassen) zeichnen sich durch hohes (und auch kontrolliert einstellbares) Molekulargewicht und die Abwesenheit (bzw. den sehr geringen Anteil) von - durch die Polymerisation erzeugten - strukturellen Defekten aus. Mit Hilfe des erfindungsgemäßen Verfahrens sind Polyarylene mit einem DP von größer 100, bevorzugt von größer 200, insbesondere 300, erhältlich.

Diese Polymere, erzeugt durch das erfindungsgemäße Verfahren, weisen damit deutliche Verbesserungen

gegenüber dem Stand der Technik auf und sind somit ebenfalls Gegenstand der Erfindung.

Wie oben beschrieben ist eine weitere bevorzugte Ausführungsform des erfindungsgemäßen Verfahrens dessen Verwendung zur Verknüpfung monofunktioneller Verbindungen.

Die dadurch erzeugten dimeren Verbindungen zeichnen sich durch die Abwesenheit (bzw. den sehr geringen Anteil) von - durch die Reaktion erzeugten - strukturellen Defekten wie z.B. Stellungsisomerien oder Hydro-dehalogenierungsprodukten, aus. Diese Verbindungen, erzeugt durch das erfindungsgemäße Verfahren, weisen damit deutliche Verbesserungen gegenüber dem Stand der Technik auf und sind somit ebenfalls Gegenstand der Erfindung. Die besondere Unempfindlichkeit des beschriebenen Verfahrens gegenüber Substitutenteneffekten, macht die Verwendung einer wesentlich größeren Zahl von Edukten möglich. Hierunter befinden sich auch Edukte mit Substitutionsmustem, welche bislang nicht mit Yamamotoreaktionen zu verknüpfen waren (z. B. zweifach ortho-substituiertes Arylhalogenid).

Ein bevorzugtes erfindungsgemäßes Verfahren (Dimerisierung oder Polymerisation) ist wie folgt zu beschreiben:

Die Reaktionsapparatur wird zunächst getrocknet, das gewählte Lösemittel (z. B. Toluol) dann entgast und mit Argon überlagert.

Die vorbereitete Katalysatormischung wird mit dem Reduktionsmittel wie z. B. Mangan vermengt und unter Argon bei z.B. 50°C gerührt.

Die Edukte (Monomere) werden in einem geeigneten Lösungsmittel wie z. B. Toluol gelöst und die Reaktion wird gestartet durch Zugabe der Eduktlösung zur Katalysatorlösung.

Gegebenenfalls werden geringe Mengen monofunktioneller Verbindungen ("Endcapper") oder tri- oder multifunktioneller Gruppen ("Verzweiger") zugesetzt.

Die Reaktion wird unter heftigem Rühren bei Reaktionstemperatur gehalten und innerhalb eines Zeitraums von ca. 48 - 120 Stunden durchgeführt.

Es hat sich als sinnvoll herausgestellt, am Ende der Reaktion ein sogenanntes End-Capping durchzuführen, d. h. monofunktionelle Verbindungen zuzugeben, die eventuelle reaktive Endgruppen in den Polymeren abfangen.

Am Ende der Reaktion kann nun das Polymer durch gängige Reinigungsverfahren, wie z. B. Ausfällen, Umfällen, Extraktion u. ä. weiter aufgereinigt werden. Für die Verwendung in hochwertigen Anwendungen (z. B. Polymere Leuchtdioden) muß i. d. R. die Verunreinigung mit organischen (z. B. Oligomeren) und anorganischen Substanzen (z. B. Ni-Reste, Reste des Reduktionsmittels) auf ein möglichst geringes Maß gebracht werden. Dies kann für Ni und metallische Reduktionsmittel auf verschiedenste Art und Weise erreicht werden, z. B. durch Ionen-Austauscher, Flüssig-Flüssig-Extraktion, Extraktion mit Komplexbildnern und anderen Verfahren, für die Entfernung von niedermolekularen Anteilen z. B. durch Fest-Flüssig- oder Flüssig-Flüssig-Extraktion oder auch mehrfaches Umfällen geschehen, für die Entfernung von weiterer anorganischer Verunreinigung z. B. durch die bereits für Nickel und die niedermolekularen Anteile beschriebenen Verfahren, aber auch durch Extraktion mit beispielsweise Mineralsäuren geschehen.

Mit dem hier beschriebenen Verfahren können nun beispielsweise Polyarylene, wie diese in EP-A-842.208, WO 00/22026, WO 00/46321, WO 99/54385, WO 00155927, WO 97/31048, WO 97/39045, WO 92/18552, WO 95/07955, EP-A-690.086 und den noch nicht offengelegten Anmeldeschriften DE 10114477.6 und DE 10143353.0 beschrieben werden, besonders effizient hergestellt werden. Häufig weisen die Polymere, hergestellt durch das erfindungsgemäße Verfahren, Vorteile gegenüber den Angaben in dieser zitierten Literatur auf, so zum Beispiel bezüglich der Defektfreiheit, des Molekulargewichts, der Molekulargewichtsverteilung und damit häufig auch bezüglich der entsprechenden Anwendungseigenschaften.

Gegenstand der Erfindung sind also weiterhin Poly(arylene), dadurch gekennzeichnet, daß sie durch oben beschriebenes Verfahren hergestellt wurden und eine besonders geringe Defektrate aufweisen, insbesondere solche, die einen Phosphorgehalt von weniger als 10 ppm aufweisen.

Die erfindungsgemäßen Polymere können in elektronischen Bauteilen, wie organische Leuchtdioden (OLEDs), organischen integrierten Schaltungen (O-ICs), organischen Feld-Effekt-Transistoren (OFETs), organischen Dünnfilmtransistoren (OTFTs), organischen Solarzellen (O-SCs), organischen Laserdioden (O-Laser), organischen Farbfilter für Liquid-Crystal-Displays oder organischen Photorezeptoren, Verwendung finden. Diese Verwendungen sind ebenfalls Bestandteil der vorliegenden Erfindung.

Die beschriebene Erfindung wird durch die Beschreibung und die nachfolgend aufgeführten Beispiele erläutert, ist aber keinesfalls auf diese beschränkt, sondern kann durch den Fachmann natürlich einfach auf die oben aufgezeigten bzw. in der zitierten Literatur beschriebenen Systeme übertragen werden.

**Beispiele des erfindungsgemäßen Verfahrens**

**Beispiel V1:** Herstellung der Katalysator-Lösung:

Mangan (0.11 g, 2 mmol) wurde mit, in DMF (5 mL) gelöstem, $NiBr_2$ (200 mg, 0.9 mmol) bei Raumtemperatur gemischt. Die Liganden-Lösung, bestehend aus 150 mg (0.96 mmol) Bipyridin, 0.12 mL (1.0 mmol) COD gelöst in 15

mL Toluol), wurde langsam zugegeben, nach 5-10 Minuten wurde die Lösung tief violett. Die Mischung wurde eine Nacht bei Raumtemperatur kräftig gerührt. Diese Lösung war einige Woche unter Argon stabil.

**A1: Herstellung von dimeren, multifunktionellen Verbindungen**

**Beispiel D1:**

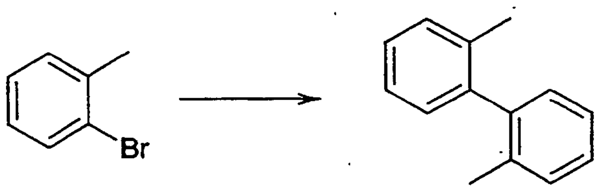

D1

Mangan (1.6 g, 30 mmol) und die Katalysatorlösung (11 mL) wurden 10 Minuten bei 50°C unter Argon gerührt. Die Mischung blieb stabil violett. Dann wurde 2-Bromtoluol (3.4 g, 20 mmol) in 50 mL Toluol zugegeben, die Reaktionsmischung wurde rot. Die Reaktionsmischung wurde 48h bei 50°C gerührt (bis die Lösung wieder violett wurde). Das Gemisch wurde dann auf Raumtemperatur abgekühlt, über Celite filtriert und einrotiert. Der Rückstand wurde in $CH_2Cl_2$ gelöst und mit HCl 1M (100 mL) und Wasser gewaschen. Die organische Phase wurde über $Na_2SO_4$ getrocknet, filtriert und einrotiert. Nach Chromatographie (Kieselgel, Eluent: Hexan), erhielt man dabei 1.7 g (Ausbeute 94%) des gewünschten Produkts (D1), welches laut HPLC eine Reinheit von 99.9 % aufwies.
$^1$H NMR ($CDCl_3$): 7.41 ppm (m, 4 H); 7.37 ppm (m, 2 H); 7.27 ppm (dd, 2 H), 2.23 ppm (s, 6 H);
HPLC: 95% MeOH/THF (9:1) +5% Wasser; 1 ml/min, Stablebond SB-C18; 3,5 μm; 4,6 x 75 mm, 40°C, UV/VIS Detektion.

**Beispiel D2:**

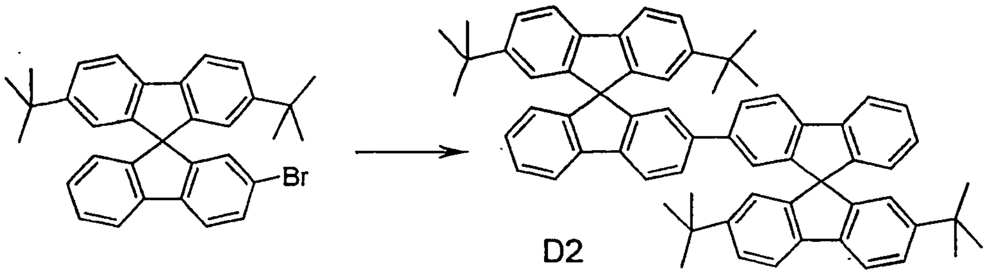

D2

*Variation 1:*

Mangan (0.3 g, 5.4 mmol) und die Katalysatorlösung (1 mL) wurden 10 Minuten bei 50°C unter Argon gerührt. Die Mischung blieb stabil violett. Dann wurde das Edukt (1.01 g, 2 mmol) in 10 mL Toluol gelöst zugegeben, die Reaktionsmischung wurde tiefrot. Die Reaktionsmischung wurde 48h bei 50°C gerührt. Das Gemisch wurde dann auf Raumtemperatur abgekühlt, über Celite filtriert und einrotiert. Der Rückstand wurde in $CH_2Cl_2$ gelöst und mit HCl 1M (100 mL) und Wasser gewaschen. Die organische Phase wurde über $Na_2SO_4$ getrocknet, filtriert und einrotiert.
Es ergaben sich 820 mg (Ausbeute 96%) des gewünschten Produkts (D2), welches laut HPLC eine Reinheit von 98 % aufwies, sowie 2% debromiertes Edukt.
$^1$H NMR ($CDCl_3$): 7.79 (2H, d, J=7.34 Hz); 7.74 (2H, d, J=7.98 Hz); 7.69 (4H, d, J=7.93 Hz); 7.30-7.38 (8H, m); 7.05 (2H, dt, J=7.91 Hz, J=1.03); 6.87 (2H, d, J=1.39 Hz); 6.67 (2H, d, J=7.61 Hz); 6.62 (4H, d, J=1.39 Hz); 1.10 (36H, s).
$^{13}$C NMR ($CDCl_3$): 150.68, 150.04, 149.91, 148.90, 141.20, 140.86, 140.66, 139.24, 127.60, 127.35, 126.72, 124.74, 124.09, 122.55, 120.61, 119.83, 119.78, 119.05, 66.29, 34.74, 31.37.

Variation 2:

Mangan (0.3 g, 5.4 mmol) und das Edukt (1.01 g, 2 mmol), gelöst in 10 ml Toluol, wurden 10 Minuten bei 50°C unter Argon gerührt. Die violette Katalysatorlösung (1 mL) wurde zugegeben. Die Reaktionsmischung wurde tiefrot. Weitere Beobachtungen analog der Variante 1.

Variation 3:

Mangan (0.33 g, 6 mmol) wurde mit, in DMF (0.5 mL) gelöstem, $NiBr_2$ (20 mg, 0.09 mmol) bei Raumtemperatur gemischt. Die Ligand-Lösung (15 mg (0.096 mmol) Bipyridyl, 0.012 mL (0.1 mmol) COD in 1.5 mL Toluol gelöst) wurde langsam zugegeben, nach 5-10 Minuten wurde die Lösung tief violett. Die Mischung wurde eine Nacht bei Raum Temperatur kräftig gerührt. Dann wurde das Edukt (2.03 g, 4 mmol) in 10 mL Toluol gelöst zugegeben, die Reaktionsmischung wurde tiefrot. Weitere Beobachtungen analog der Variante 1.

**Beispiel D3:**

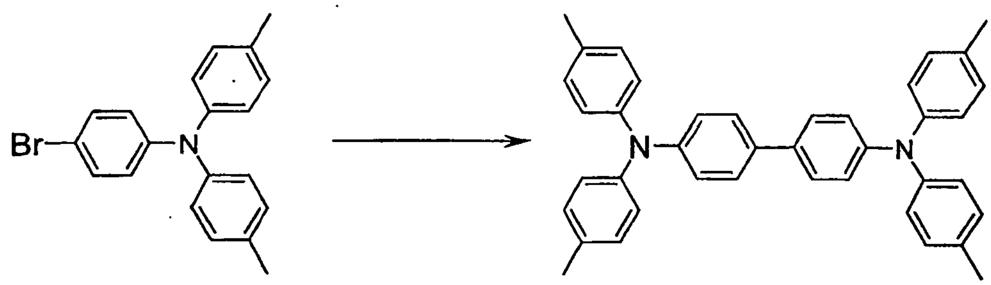

D3

Mangan (1.04 g, 18.9 mmol) und die Katalysatorlösung (8.75 mL) wurden 10 Minuten bei 50°C unter Argon gerührt. Die Mischung blieb stabil violett. Dann wurde das Edukt (4.54 g, 12.9 mmol) in 35 mL Toluol gelöst zugegeben, die Reaktionsmischung wurde tiefrot. Die Reaktionsmischung wurde 48h bei 50°C gerührt. Das Gemisch wurde dann auf Raumtemperatur abgekühlt, über Celite filtriert und einrotiert. Der Rückstand wurde in $CH_2Cl_2$ gelöst und mit HCl 1M (100 mL) und Wasser gewaschen. Die organische Phase wurde über $Na_2SO_4$ getrocknet, filtriert und einrotiert. Das Produkt wurde aus Ethanol umkristallisiert.
*Es ergaben sich 3.16* g *(Ausbeute 90%) des gewünschten Produkts (**D3**), welches laut HPLC eine Reinheit von 99.9 % aufwies.*
$^1$H NMR ($CDCl_3$): 7.37 (4H, m); 7.20-6.90 (20H, m); 2.3 (12H, s).

**A2: Herstellung von Polymeren**

Die Herstellung entsprechender Monomere ist u. a. in den o. g. nicht offengelegten Anmeldeschriften DE 10114477.6 und DE 10143353.0 ausgeführt; diese werden hiermit via Zitat als Bestandteil der vorliegenden Erfindung betrachtet.

**Beispiel P1:** *Copolymerisation von 50 mol% 2,7-Dibrom-2;3',6',7'-tetra(2-methylbutyloxy)spirobifluoren (**M1**), 40 mol% 2,7-Dibrom-9-(2',5'-dimethyl-phenyl)-9-[3",4"-bis(2-methyl-butyloxy)phenyl]fluoren (**M2**), 10% N,N'-Bis (4-bromphenyl)-N,N'-bis (4-tert-butylphenyl)benzidin (**M3**) (**Polymer P1**).*

Mangan (440 mg, 8mmol) und die Katalysator Lösung (2 mL) wurden 10 Minuten bei 50°C unter Argon gerührt. Die Mischung blieb stabil violett. Dann wurden die Monomere (655 mg (0.8 mmol) M1, 433 mg (0.64 mmol) M2, 121mg (0.16 mmol) M3) in 20 mL Toluol gelöst zugegeben, die Reaktionsmischung wurde rot. Die Reaktionsmischung wurde 5 Tage bei 50°C gerührt. Das Gemisch wurde dann auf Raumtemperatur abgekühlt, mit 10 mL Toluol verdünnt und über Celite filtriert. Die organische Phase wurde 3x mit HCl (50 mL) und mit $H_2O$ gewaschen und durch Eintropfen in 500 mL Methanol.gefällt. Das Polymer wurde in 50 mL Toluol gelöst, mit 500 mL MeOH ausgefällt, gewaschen und im Vakuum getrocknet. Das Polymer wurde 48h in einem Gemisch aus THF/MeOH 1/1 soxhletiert. Das Polymer wurde in 50 mL Toluol gelöst und in 500 mL Methanol ein weiteres Mal umgefällt, abgesaugt und bis zur Massenkonstanz getrocknet. Man erhielt 0.8 g (Ausbeute 84%) des Polymers **P1** als Feststoff.
$^1$H NMR ($CDCl_3$): 7.8-7.7 (m, 1 H, Spiro); 7.7-7.1 (m, 10.7 H, Fluoren, Spiro, TAD); 6.6 (br. s, 0.8H, Fluoren), 6.21 (m,

1 H, Spiro); 4.0-3.4 (3 x m, 5.6 H, $OCH_2$), 2.16 (s, 1.2 H, $CH_3$); 1.9-0.7 (m, Alkyl H, darunter bei 1.30 t-Butyl).
GPC: THF; 1 ml/min, Plgel 10μm Mixed-B 2 x 300 x 7.5 $mm^2$, 35°C, RI Detektion:

Mw = 276400 g/mol, Mn = 73500 g/mol.
Es wurden auch die Restgehalte an Metallen via ICP-AES-MS bestimmt:
Nickel < 3 ppm, Mangan < 5 ppm (jeweils unter der Nachweisgrenze).

Weitere Polymere wurden analog den Beschreibungen für **P1** dargestellt. Die Molekulargewichte $M_w$ und $M_n$ sind in der folgenden Tabelle zusammengefaßt:

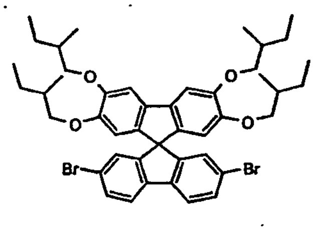

M1

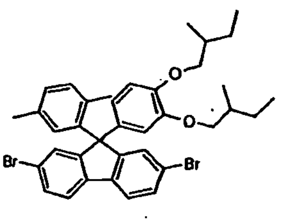

M2

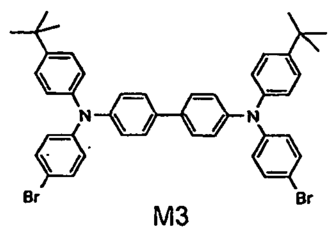

M3

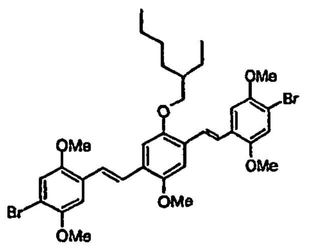

M4

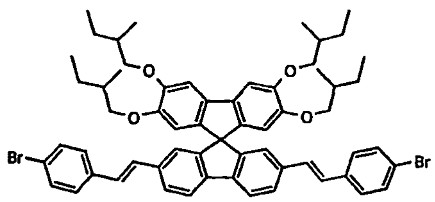

M5

| | Anteil der Monomere in der Polymerisation [%] | | | | | GPC* | |
|---|---|---|---|---|---|---|---|
| Polymer | M1 | M2 | M3 | M4 | M5 | $M_W$ (1000 g/mol) | $M_N$ (1000 g/mol) |
| P1 | 50 | 40 | 10 | | | 276 | 73 |
| P2 | 70 | | 10 | | 20 | 738 | 66 |
| P3 | | 80 | 10 | | 10 | 245 | 75 |
| P4 | | 70 | 10 | 20 | | 559 | 165 |
| P5 | 50 | | | 50 | | 270 | 55 |
| *GPC: THF; 1 ml/min, Plgel 10pm Mixed-B 2 x 300 x 7.5 $mm^2$, 35°C, RI Detektion | | | | | | | |

**Patentansprüche**

1. Verfahren zur Kupplung aromatischer oder heteroaromatischer Halogenverbindungen unter Bildung einer oder mehrerer C-C-Einfächbindung, **dadurch gekennzeichnet, daß** ein Ni-(0)-Komplex, welcher mindestens zwei verschiedenartige Liganden enthält, wobei jeweils mindestens die Ligand aus jeder der beiden Gruppen der heteroatom-haltigen Liganden (Gruppe 1) und der π-System Liganden (Gruppe 2) gewählt werden in katalytischen Mengen verwendet wird,
und ein Reduktionsmittel zum Einsatz kommt, welches verbrauchtes Nickel wieder in Ni-(0) überführt;
die Reaktion in einem wasserfreien, aprotischem Medium unter weitestgehend inerter Atmosphäre stattfindet,
mit der Maßgabe, daß dabei keine Phosphor-haltige Verbindung zugesetzt wird.

2. Verfahren gemäß Anspruch 1 **dadurch gekennzeichnet, daß** es einphasig abläuft.

3. Verfahren gemäß Anspruch 1 und/oder 2 **dadurch gekennzeichnet, daß** die aromatischen oder heteroaromatischen Halogenverbindungen Aromaten oder. Heteroaromaten, mit 2 bis 40 C-Atomen, welche mit einem oder auch mehreren linearen, verzweigten oder cyclischen Alkyl- bzw. Alkoxyresten mit 1 bis 20 C-Atomen, bei denen wiederum eine oder mehrere $CH_2$-Gruppen, die nicht aufeinander folgen, durch O, C=O, oder eine Carboxygruppe ersetzt sein können, substituierten oder unsubstituierten C-2 bis C-20 Aryl- oder Heteroarylresten, Fluor, Cyano-, Nitrogruppen substituiert.sein können bzw. auch unsubstituiert sein können, bedeuten.

4. Verfahren gemäß Anspruch 3 **dadurch gekennzeichnet, daß** die Aromaten oder Heteroaromaten substituierten oder unsubstituierten Derivaten von Benzol, Naphthalin, Anthracen, Pyren, Biphenyl, Fluoren, Spiro-9,9'-bifluoren, Phenanthren, Perylen, Chrysen, Naphthacen, Pentacen, Triptycen, Pyridin, Furan, Thiophen, Benzothiadiazol, Pyrrol, Chinolin, Chinoxalin, Pyrimidin oder Pyrazin entsprechen.

5. Verfahren gemäß einem oder mehreren der Ansprüche 1 bis 4 **dadurch gekennzeichnet, daß** der Katalysator vorab hergestellt wird.

6. Verfahren gemäß einem oder mehreren der Ansprüche 1 bis 4 **dadurch gekennzeichnet, daß** der Katalysator in-situ hergestellt wird.

7. Verfahren zur Herstellung eines Ni-(0)-Komplexes gemäß Anspruch 1, **dadurch gekennzeichnet, daß** ein Reduktionsmittel mit in DMF gelösten Ni-(II)-salz bei Raumtemperatur gemischt wird, eine toluolische Liganden-Lösung langsam zugegeben wird, und diese kräftig gerührt wird.

8. Verfahren gemäß einem oder mehreren der Ansprüche 1 bis 6 **dadurch gekennzeichnet, daß** die Liganden der Gruppe 1 Heteroatome aus der 5. oder 6. Hauptgruppe, unter Ausnahme von Phosphor, enthalten.

9. Verfahren gemäß Anspruch 8 **dadurch gekennzeichnet, daß** die Liganden Stickstoff und/oder Sauerstoff enthalten.

10. Verfahren gemäß den Ansprüchen 8 und/oder 9, **dadurch gekennzeichnet, daß** die Liganden zwei $\eta^1$-Koordinationen zum Nickel aufweisen, die dabei jeweils über die Heteroatome verlaufen.

11. Verfahren gemäß einem oder mehreren der Ansprüche 1 bis 6 und 8 bis 10, **dadurch gekennzeichnet, daß** die Liganden der Gruppe 2 mindestens eine über ein $\pi$-System verlaufende $\eta^2$-Koordination zum Nickel aufweisen.

12. Verfahren gemäß Anspruch 11 **dadurch gekennzeichnet, daß** diese Liganden Alkin- oder Alkengruppen enthalten.

13. Verfahren gemäß den Ansprüchen 11 und/oder 12, **dadurch gekennzeichnet, daß** diese Liganden zwei $\eta^2$-Koordinationen zum Nickel aufweisen, die dabei jeweils über die $\pi$-Systeme verlaufen.

14. Verfahren gemäß einem oder mehreren der Ansprüche 1 bis 6 und 8 bis 13, **dadurch gekennzeichnet, daß** als Lösungsmittel weniger polare Lösungsmitteln wie z. B. aliphatische und aromatische Kohlenwasserstoffen dienen.

15. Verfahren gemäß Anspruch 14 **dadurch gekennzeichnet, daß** als Lösungsmittel Pentan, Cyclohexen, Toluol oder Xylol dienen.

16. Verfahren gemäß den Ansprüchen 14 und/oder 15, **dadurch gekennzeichnet, daß** diese Lösemittel mit inerten, dipolaren Lösungsmitteln wie N,N'-Dimethylformamid, N,N'-Dimethylacetamid, N-Methylpyrrolidin-2-on, Tetramethylharnstoff, Dimethylsulfoxid oder Sulfolan gemischt werden.

17. Verfahren gemäß Anspruch 16, **dadurch gekennzeichnet, daß** ein Gemisch von DMF und Toluol verwendet wird.

18. Polyarylene mit einem Phosphorgehalt von weniger als 10ppm, erhältlich durch ein Verfahren gemäß einem oder mehreren der Ansprüche 1 bis 6 und 8 bis 17.

## Claims

1. A process for coupling aromatic or heteroaromatic halogen compounds to form one or more C-C single bonds, **characterized in that** an Ni(0) complex comprising at least two different ligands, with at least one ligand being selected from each of the two groups consisting of heteroatom-containing ligands (group 1) and of $\pi$ system ligands (group 2), is used in catalytic amounts,
and a reducing agent which converts consumed nickel back into Ni(0) is used; the reaction takes place in an anhydrous, aprotic medium under a very largely inert atmosphere,
with the proviso that no phosphorus-containing compound is added.

2. The process as claimed in claim 1, **characterized in that** it occurs in a single phase.

3. The process as claimed in claim 1 and/or 2, **characterized in that** the aromatic or heteroaromatic halogen compounds are aromatics or heteroaromatics having from 2 to 40 carbon atoms, which can be substituted by one or more linear, branched or cyclic alkyl or alkoxy radicals which have from 1 to 20 carbon atoms and in which one or more nonadjacent $CH_2$ groups can be replaced by O, C=O or a carboxy group, substituted or unsubstituted C2-C20-aryl or -heteroaryl radicals, fluorine, cyano, nitro groups or can also be unsubstituted.

4. The process as claimed in claim 3, **characterized in that** the aromatics or heteroaromatics are substituted or unsubstituted derivatives of benzene, naphthalene, anthracene, pyrene, biphenyl, fluorene, spiro, 9,9'-bifluorene, phenanthrene, perylene, chrysene, naphthacene, pentacene, triptycene, pyridine, furan, thiophene, benzothiadiazole, pyrrole, quinoline, quinoxaline, pyrimidine or pyrazine.

5. The process as claimed in one or more of claims 1 to 4, **characterized in that** the catalyst is prepared beforehand.

6. The process as claimed in one or more of claims 1 to 4, **characterized in that** the catalyst is prepared in situ.

7. A process for preparing an Ni(0) complex as claimed in claim 1, **characterized in that** a reducing agent is mixed with an Ni(II) salt dissolved in DMF at room temperature, a ligand solution in toluene is slowly added and the mixture is stirred vigorously.

8. The process as claimed in one or more of claims 1 to 6, **characterized in that** the ligands of group 1 contain heteroatoms from main group 5 or 6, with the exception of phosphorus.

9. The process as claimed in claim 8, **characterized in that** the ligands contain nitrogen and/or oxygen.

10. The process as claimed in claim 8 and/or 9, **characterized in that** the ligands have two $\eta^1$ coordinations to the nickel, in each case via the heteroatoms.

11. The process as claimed in one or more of claims 1 to 6 and 8 to 10, **characterized in that** the ligands of group 2 have at least one $\eta^2$ coordination via a $\pi$ system to the nickel.

12. The process as claimed in claim 11, **characterized in that** these ligands comprise alkyne or alkene groups.

13. The process as claimed in claim 11 and/or 12, **characterized in that** these ligands have two $\eta^2$ coordinations to the nickel, in each case via the $\pi$ systems.

14. The process as claimed in one or more of claims 1 to 6 and 8 to 13, wherein relatively nonpolar solvents such as aliphatic and aromatic hydrocarbons serve as solvent.

15. The process as claimed in claim 14, **characterized in that** pentane, cyclohexene, toluene or xylene serve as solvent.

16. The process as claimed in claim 14 and/or 15, **characterized in that** these solvents are mixed with inert, dipolar solvents such as N,N'-dimethylformamide, N,N'-dimethylacetamide, N-methylpyrrolidin-2-one, tetramethylurea, dimethyl sulfoxide or sulfolane.

17. The process as claimed in claim 16, **characterized in that** a mixture of DMF and toluene is used.

**18.** A polyarylene which has a phosphorus content of less than 10 ppm and is obtainable by a process as claimed in one or more of claims 1 to 6 and 8 to 17.

## Revendications

**1.** Procédé destiné au couplage de composés halogénés aromatiques ou hétéroaromatiques en formant une ou plusieurs liaisons C-C simples, **caractérisé en ce qu'**un complexe Ni-(0), lequel contient au moins deux ligands de nature différente, dont au moins un ligand étant choisi dans chacun des deux groupes des ligands contenant un hétéroatome (groupe 1) et des ligands du système $\pi$ (groupe 2), est employé dans des quantités catalytiques,
et un réducteur est employé, lequel réduit le nickel épuisé à nouveau en Ni-(0); la réaction a lieu dans un milieu aprotique non aqueux, sous atmosphère largement inerte,
ce faisant, par convention, aucun composé contenant du phosphore n'est ajouté.

**2.** Procédé selon la revendication 1, **caractérisé en ce qu'**il se déroule en une phase.

**3.** Procédé selon la revendication 1 et/ou 2, **caractérisé en ce que** les composés halogénés aromatiques ou hétéroaromatiques sont des aromates ou des hétéroaromates ayant 2 à 40 atomes C, lesquels peuvent être substitués ou aussi non substitués à un ou aussi plusieurs groupes alcoxyles ou groupes alkyles linéaires, ramifiés ou cycliques ayant 1 à 20 atomes C, dans lesquels à leur tour un ou plusieurs groupes $CH_2$ qui ne se suivent pas peuvent être remplacés par O, C=O, ou un groupe carboxyle, à des groupes aryles ou des groupes hétéroaryles C-2 à C-20 substitués ou non substitués, à des groupes fluor, des groupes cyano ou à des groupes nitro.

**4.** Procédé selon la revendication 3, **caractérisé en ce que** les aromates ou les hétéroaromates correspondent à des dérivés substitués ou non substitués de benzène, naphtalène, anthracène, pyrène, biphénylé, fluorène, bifluorène spiro 9,9', phénanthrène, pérylène, chrysène, naphtacène, pentacène, triptycène, pyridine, furane, thiophène, benzothiadiazol, pyrrol, quinoléine, quinoxaline, pyrimidine ou pyrazine.

**5.** Procédé selon l'une ou plusieurs des revendications 1 à 4, **caractérisé en ce que** le catalyseur est préparé en premier.

**6.** Procédé selon l'une ou plusieurs des revendications 1 à 4, **caractérisé en ce que** le catalyseur est préparé in situ.

**7.** Procédé destiné à la préparation d'un complexe Ni-(0) selon la revendication 1, **caractérisé en ce qu'**un réducteur est mélangé à température ambiante avec du sel de nickel (II) dissout dans du DMF, une solution de ligands toluylique est ajoutée lentement et celle-ci est agitée énergiquement.

**8.** Procédé selon l'une ou plusieurs des revendications 1 à 6, **caractérisé en ce que** les ligands du groupe 1 contiennent des hétéroatomes du 5$^{ème}$ ou du 6$^{ème}$ groupe principal, à l'exception du phosphore.

**9.** Procédé selon la revendication 8, **caractérisé en ce que** les ligands contiennent de l'azote et/ou de l'oxygène.

**10.** Procédé selon les revendications 8 et/ou 9, **caractérisé en ce que** les ligands présentent deux coordinations $\eta^1$ au nickel qui passent chacune respectivement par les hétéroatomes.

**11.** Procédé selon l'une ou plusieurs des revendications 1 à 6 et 8 à 10, **caractérisé en ce que** les ligands du groupe 2 présentent au moins une coordination $\eta^2$ au nickel qui passe par un système $\pi$.

**12.** Procédé selon la revendication 11, **caractérisé en ce que** ces ligands contiennent des groupes alcynes ou des groupes alcènes.

**13.** Procédé selon les revendictions 11 et/ou 12, **caractérisé en ce que** ces ligands présentent deux coordinations $\eta^2$ au nickel qui passent chacune respectivement par les systèmes $\pi$.

**14.** Procédé selon l'une ou plusieurs des revendications 1 à 6 et 8 à 13, **caractérisé en ce que** les solvants moins polaires tels que par exemple les hydrocarbures aliphatiques ou aromatiques servent de solvants.

**15.** Procédé selon la revendication 14, **caractérisé en ce que** le pentane, le cyclohexane, le toluène ou le xylène

servent de solvant.

**16.** Procédé selon les revendications 14 et/ou 15, **caractérisé en ce que** ces solvants sont mélangés à des solvants dipolaires inertes tels que le N,N'-diméthylformamide, le N,N'-diméthylacétamide, la N-méthylpyrrolidine-2-one, la tétraméthylurée, le diméthylsulfoxyde ou le sulfolane.

**17.** Procédé selon la revendication 16, **caractérisé en ce qu'**un mélange de DMF et de toluène est utilisé.

**18.** Polyarylènes avec une teneur en phosphore de moins de 10ppm pouvant être obtenus par un procédé selon l'une ou plusieurs des revendications 1 à 6 et 8 à 17.